(19) **Europäisches Patentamt**
**European Patent Office**
**Office européen des brevets**

(11) **EP 1 119 284 B1**

(12) **EUROPEAN PATENT SPECIFICATION**

(45) Date of publication and mention
of the grant of the patent:
**16.02.2011 Bulletin 2011/07**

(51) Int Cl.:
***A61B 3/103*** *(2006.01)*

(21) Application number: **99951829.3**

(86) International application number:
**PCT/US1999/023327**

(22) Date of filing: **07.10.1999**

(87) International publication number:
**WO 2000/019885 (13.04.2000 Gazette 2000/15)**

(54) **DEVICE FOR MEASURING ABERRATION REFRACTION OF THE EYE**

VORRICHTUNG ZUR MESSUNG DER BRECHUNG DER ABERRATION DES AUGES

DISPOSITIF POUR MESURER LA REFRACTION D'ABERRATION OCULAIRE

(84) Designated Contracting States:
**AT BE CH CY DE DK ES FI FR GB GR IE IT LI LU MC NL PT SE**

(30) Priority: **07.10.1998 UA 98105286**

(43) Date of publication of application:
**01.08.2001 Bulletin 2001/31**

(73) Proprietor: **Tracey Technologies, L.L.C.**
**Bellaire, TX 77401 (US)**

(72) Inventors:
- **MOLEBNY, Vasyl**
  **Kiev 252028 (UA)**
- **PALLIKARIS, Ioannis**
  **GR-Heraklion (GR)**
- **CHYZH, Igor**
  **Kiev 252217 (UA)**
- **SOKURENKO, Vyacheslav**
  **Kiev 252070 (UA)**
- **NAOUMIDIS, Leonidas**
  **GR-Heraklion (GR)**
- **WAKIL, Joussef**
  **Houston, TX 77005 (US)**

(74) Representative: **Wilkinson, Stephen John et al**
**Stevens, Hewlett & Perkins**
**1 St. Augustine's Place**
**Bristol BS1 4UD (GB)**

(56) References cited:
**US-A- 4 609 287    US-A- 5 148 205**
**US-A- 5 258 791    US-A- 5 581 405**

- **Journal of the Optical Society of America A (Optics, Image Science and Vision) Opt. Soc. America USA, vol. 15, no. 9, September 1998 (1998-09), pages 2449-2456, XP002403895 ISSN: 0740-3232**
- **NAVARRO R ET AL: "Monochromatic aberrations and point-spread functions of the human eye across the visual field" JOURNAL OF THE OPTICAL SOCIETY OF AMERICA. A, OPTICS AND IMAGE SCIENCE, vol. 15, no. 9, September 1998 (1998-09), pages 2522-2529, XP002311517 ISSN: 0740-3232**

## Description

### TECHNICAL FIELD

The present invention relates to medical ophthalmological equipment, more specifically, it relates to devices for measuring the refraction of the eye as a function of spatial pupil coordinates.

### BACKGROUND OF THE INVENTION

Measuring devices are known for the study of the refraction component of the optical system of the eye, which depend on spatial pupil coordinates. These include M. S. Smimov's device for measuring the wave aberration [1], Van den Brink's device for measuring the transverse aberration [2], M. M. Sergienko's device for measuring the physiological astigmatism [3], and a spatially resolved refractometer [4]. The above devices, based on Scheiner's principle, involve point-by-point investigation over utilizing a number of optical techniques. However, in using all such devices the direct participation of the patient is needed in the preliminary aligning of the eye and in the aberration measurements.

Major disadvantages of the above measuring devices are their low accuracy and productivity, a prolonged measurement process resulting in the patient's fatigue, variations in accommodation, and eye movements while taking measurements, thereby increasing the aberration measurement errors.

More advanced measuring devices are known which do not require the patient to act as a link in the "measurement chain". These include a device for measuring the aberration by the Foucault's knife method [5], a device for measuring the wave aberration using Hartmann-Shack sensors [6-8], and a measuring device incorporating adaptive optics that makes it possible to measure the wave aberration by means of its complete compensation [9].

A common disadvantage of the measuring devices incorporating a Hartmann-Shack sensor is the fixed field of view of the raster photoelectric analyzer of transverse-aberrations due to the mechanically rigid construction of the lens raster and the invariable mutual spatial arrangement of the photosensitive elements of the charged coupled device or "CCD" camera. This results in a fixed configuration of grid sites at the pupil plane in which aberrations are measured, thus making it impossible to obtain flexible reconfiguring of these grid sites for more detailed measurements in separate zones of the pupil depending on their aberration properties.

Other disadvantages of existing devices include: they do not incorporate means for providing an accurate reproducible "linkage" of the patient's eye to the spatial co-ordinates of the measuring device; they do not incorporate a means for adjusting the accommodation of the patient's eye that is necessary for studying the dependence of aberrations on the accommodation characteristics; and they are not capable of taking measurements on a dilated pupil without using medicines.

Refraction can also be measured using a spatially resolved objective autorefractometer as disclosed in U.S. Patent No. 5,258,791 [10]. This device provides spatially resolved refraction data using a closed measuring loop which includes a reference pattern and a measurement beam. In this device, an origin of coordinates of the detector coincides with the center of the fovea image and the detector functions as a zero-position sensor.

The spatially resolved objective autorefractometer disclosed in U.S. Patent No. 5,258,791, preferably using laser ray tracing, has a number of substantial problems relating to performance in the following basic and auxiliary functions: preliminary alignment of the optical axis of the device relative to the visual axis of the eye; accommodation monitoring of the patient's eye; allocation of points within the pupil at which refraction is measured; and measurement of the angle of laser beam incidence into the patient's eye. Respective to the above basic and auxiliary functions, the above drawbacks are inherent to the device disclosed in U.S. Patent No. 5,258,791.

Preliminary alignment of the optical axis of prior art devices relative to the visual axis of the eye may be problematic for at least the following reasons. First, the visual axis of the eye is assumed to be the line passing through the geometric center of the pupil and the fovea. However, it is known that the geometric center of the pupil does not always coincide with the visual axis due to the misalignment of the pupil opening and the optical axis of the cornea and the crystalline lens. In addition, the pupil may not be symmetrical.

Second, in prior art devices in which alignment is dependant on fixation of the patient's gaze at a focal point, changes in the position of the point at which the patient's gaze is fixed results in angular movement of the patient's eye which disturbs the previous alignment. Consequently, both points (on the pupil and on the retina) through which the center line passes do not have a definite location.

Third, the focal points in devices without ametropia compensation can clearly be observed only with an emmetropic or normal eye. When the patient's eye is ametropic, such devices will see a diffused laser beam spot whose width increases with the ametropy. It is obvious that under such conditions the gaze cannot be fixed accurately in a certain direction, which is another factor preventing an accurate alignment. Another drawback of prior art refractometers is that the fovea and the photosensitive surface of the photodetector are optically coupled by the lenses only in the emmetropic or normal eye. In the event of an ametropic eye, the decentering or defocusing of the fovea image on the above-mentioned surface of the photoelectric detector causes additional refraction measurement errors which are not compensated for. The present invention is designed to compensate for this.

Fourth, a sufficiently bright laser radiation may irritate the fovea to such a degree that the eye begins to narrow its pupil reflexly. Therefore, before performance of the eye centering procedure, medicines paralyzing the ciliar body muscles are likely to be required, which changes the refractive properties of the eye as compared with its normal natural state.

The need for accommodation monitoring of the patient's eye has not been satisfied in prior art devices. As a consequence, the patient's eye can be accommodating at any distance. It is known that the refractive properties of the eye depend on the accommodation distance. Because the accommodation is unknown to the operator, it is impossible to correlate the refraction map and the eye accommodation. It has become apparent to the present applicants that a spatially resolved refractometer should preferably include a device for adjusting to the patient's eye accommodation.

Prior art devices using electromechanical actuators greatly reduce the possibility of ensuring a high-speed scanning of the pupil and the possibility of shortening the duration of the ocular refraction measurement process.

In prior art devices using a disc or movable aperture bearing planar surface to control laser targeting, the aperture occupies only a small portion of the zone in which the laser beam intersects the planar surface. Thus, only that portion of the laser beam which is equal to the ratio of the area of one refraction measurement zone on the pupil to the entire pupil area passes through the aperture. Such a vignetting of the laser beam results in an uneconomical use of laser radiation and should be considered a major drawback of such designs.

Drawbacks in the measurement of the angle of laser beam incidence at which it crosses the necessary measurement zone of the pupil and the center of the fovea are inherent to designs which use the photodetector as a zero sensor (sensor of non-coincidence of the center of the light spot on the retina with the center of the fovea) and the acousto-optic deflector as an actuator in the feedback loop of the servo system of the laser beam angular-position measuring device because such arrangements do not provide a sufficiently high refraction measurement speed. In such designs, the time of measuring the refraction at 10 measurement points of the pupil is up to one minute. During this period the patient's eye can move up to 100 times and change its angular position due to natural tremor, "jumps" and drift.

Systematic instrument errors have plagued prior aberration refractometers. Due to an irregular distribution of the light irradiance within the light spot on the retina, unequal photosensitivity across the surface photoelectric detector, time instability of the gain of preamplifiers connected to the photoelectric detector elements, and the presence of unsuppressed glares and background illumination from the photodetector, the photodetector does not register a "zero" position of the spot on the fovea without systematic errors. Further, as a result of its own aberrations, the optical system providing for eye ray tracing contributes an angular aberration to the laser beam position. The present instrument incorporates structural elements which compensate for such an errors and thus increase the refraction measurement accuracy.

What is needed is an improved electro-optical ray tracing aberration refractometer which makes it possible to achieve the following goals: flexibility of allocation of measuring points within the pupil and to pupil and improvement in the effectiveness of using lasers by reducing the vignetting of the laser beam at the aperture diaphragm; reduction in the duration of measuring refraction across the entire pupil to around 10 - 20 milliseconds; ensuring optical coupling of the photosensitive surface of the photodetector with the fovea even for ametropic eyes as well as for accommodation monitoring at any given distance; reduction in instrument errors when measuring aberration refraction; enhancement of the accuracy and definitiveness of instrument positioning relative to the patient's eye; the potential for automation of the positioning and controllability of the working distance between the patient's eye and the device components; and enablement of instrument positioning without medically dilating the pupil. The present invention provides the aforementioned solutions and innovations.

## SUMMARY OF THE INVENTION

The object of the present invention is to provide an improved laser device for measuring the aberration refraction of the eye as defined by the appended claims. This aberration refractometer allows estimates of the ametropy, astigmatism characteristics and visual acuity and, if necessary, allows calculations of the part of the cornea to be removed by photorefractive keratectomy [11].

In a preferred embodiment the aberration refractometer comprises a light radiation source, preferably laser light or other polarized light; a telescopic system; a two-coordinate deflector consisting of two single-coordinate deflectors; a deflection angle control unit; an aperture stop; a field stop; a collimating lens; an interferential polarizing beam splitter; a position-sensitive photodetector with an objective lens; and a data processing and display unit consisting of a computer, an analog-to-digital converter and a preamplifier. Use of laser or polarized light as a probing beam in combination with the interferential beam splitter allows the polarized light to be separated from the non-polarized light reflected back from the retina and prevents it being detected by the photodetector.

The instrument of the present invention is able to reduce the time needed for measuring the refraction, eliminate light beam energy losses at the aperture stop and create a flexible system for locating the measurement points on the pupil by providing the following: the telescopic system is positioned in the probing beam path after the two-coordinate deflector at a distance corresponding to the coincidence of the entrance pupil of the telescopic system and the gap or zone between the single-coordinate deflectors, the aperture stop or diaphragm is placed between the lenses of the telescopic system at the point of coincidence of their foci, and the field stop or diaphragm is positioned in the plane of the exit pupil of the telescopic system and, at the same time, at the location of the front focus point of the collimating lens situated in front of the interferential polarizing beam splitter at such a distance from the patient's eye which is approximately equal to the focal distance of the collimating lens.

To ensure a constant optical coupling of the photosensitive surface of the photodetector and the retina for both emmetropic and ametropic eyes, a group of lenses with variable optical power is installed between the interferential polarizing beam splitter and the eye, said group of lenses having the function to adjustably form the retinal image of an ametropic eye at infinity regardless of the emmetropic or ametropic condition of the eye. The photosensitive surface of the photodetector is conjugated with the front focal plane of the objective lens and is inserted following the interferential polarizing beam splitter on the path of the light scattered by the retina.

To provide for fixation of the patient's line of sight along the optical axis of the instrument and to compensate for accommodation of the eye at the required distance while keeping constant optical conjugation of the patient's eye with the photosensitive surface of the detector, a second beam splitter or an optical axis rotation mirror, as well as a plate with a gaze fixing test pattern or a test-target for sight fixation, are optically coupled with the photosensitive surface of the photodetector and are located between the photodetector and the objective lens. A second optical group of lenses, with variable negative optical power and which function to form an image for the patient's eye of the test-target at a distance corresponding to the preset accommodation, is positioned between the second beam splitter or optical axis rotation mirror and the interferential polarizing beam splitter. When an optical axis rotation mirror is used, it is mounted on a movable base making it possible to displace the mirror to enable the light radiation scattered by the retina to reach the photodetector during the measurement of the patient's eye characteristics.

In one embodiment, to account for systematic refraction measurement errors, a second mirror, for bending or redirecting the optical axis of the probing laser beam, is inserted in the laser beam path after the last optical element before entering the patient's eye. Following the second mirror, an optical calibration unit for simulating an eye is inserted. The optical calibration unit includes an axially movable or stationary retina simulator whose optical characteristics are equivalent to those of the human retina. The second optical axis bending mirror is installed on a movable base so that it can be moved into the probing laser beam path during measurement with the optical calibration unit and moved out when measuring the patient's eye refraction.

To align the instrument relative to the patient's eye as well as to enhance accuracy and enable automation of the aligning process, the instrument is provided with a third beam splitter to insert a channel for eye alignment verification of the instrument and the patient's eye. In a preferred embodiment, the co-axial verification channel comprises one or more point of light sources and a TV or electro-optic detecting device, together serving to display the pupil and/or eye image and providing a permission channel to measure eye characteristics when the optical axis of the instrument and the visual axis of the patient's eye coincide. To enable the instrument to be used without dilating the pupil with a medicine, a laser radiation source and/or infrared light sources are incorporated into the coaxial verification alignment mechanism. It is contemplated that the instrument can be used to make refraction measurements under conditions simulating either day or night light conditions.

In an alternate embodiments it is further contemplated that the alignment verification can be done under the control of the operator of the device or can be automated. In one embodiment, the co-axial verification channel provides either visual or acoustic notification that coincidence between the instrument optical axis and the patient's visual axis is proximate or near to "on target" status. Once this status is attained, the instrument is "armed" electronically. Once full coincidence is attained, the measurement controller automatically causes spatially defined parallel light beams, preferably laser beams, to be rapidly fired and to enter the eye through the input channel. Light reflecting from the retina is directed to the retinal spot detecting channel for spatial and intensity characterization. This process can permit upwards of at least 5 replicate measurements over 65 spatial locations to be taken within 15 milliseconds without the need for the patient to actively participate in the targeting and alignment process.

## BRIEF DESCRIPTION OF THE DRAWINGS

For a more complete understanding of the present invention, including features and advantages, reference is now made to the detailed description of the invention along with the accompanying figures:

Figure 1: Functional schematic diagram of instrument for measuring aberration refraction.
Figure 2: Schematic illustration of the operation of the device for measuring the transverse aberration of the laser beam on the eye retina.

Figure 3: Schematic illustration of the principle of operation of the means for positioning the measuring device in relation to the patient's eye.

Figure 4: An example of a map showing the location of ocular refraction measurement points, constructed with the aid of a computer.

Figure 5: General perspective view of a prototype device for measuring the aberration refraction.

Figure 6: An example of a map showing the aberration refraction of the live eye, constructed with the eye aberration refractometer of the subject invention.

Figure 7: Schematic illustration of photodetection using linear array detectors.

Figure 8: Schematic diagram of an alternative embodiment of an aberration refractometer according to certain aspects of the present invention;

## DETAILED DISCLOSURE OF THE INVENTION

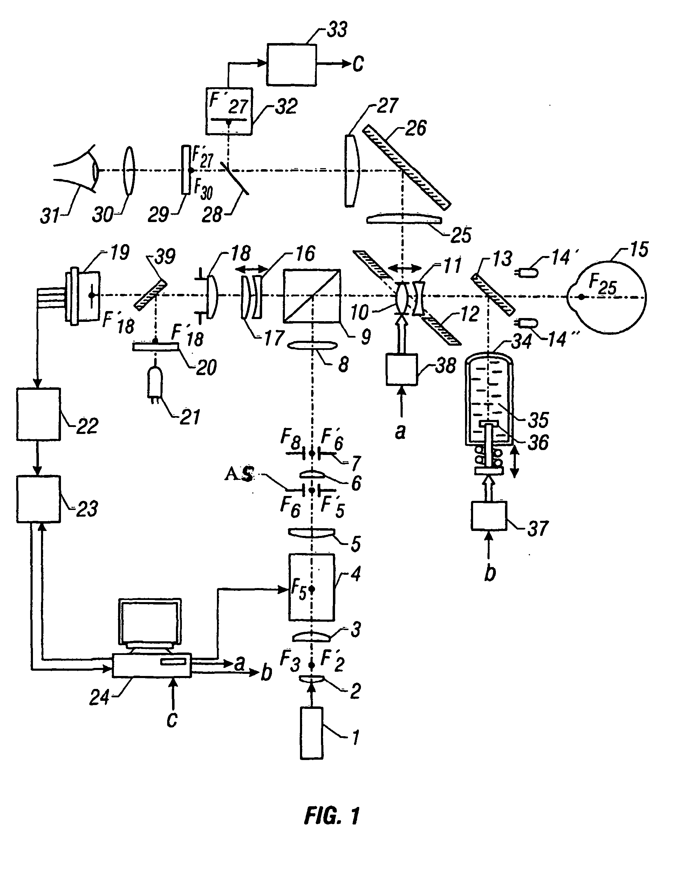

The instrument described herein was developed to provide an aberration refractometer able to accurately and quickly provide a refractive map of either emmetropic or ametropic eyes without accommodation error. Figure 1 shows a functional diagram of a preferred embodiment of the subject instrument. A light source whose radiation is used for the ray tracing of the eye is provided, as for example, by laser 1. A telescopic expander comprising for example lenses 2 and 3 provides a normal functioning of a two-coordinate acousto-optic deflector 4 preferably consisting of two single-coordinate deflectors. A telescopic laser-beam narrower is formed by lenses 5 and 6 with an aperture stop or diaphragm AS located at the common foci of the lenses 5 and 6. A field stop or diaphragm 7 is placed at the back focus of lens 6 so that its image formed by the telescopic narrower in the back-pass is located between the single-coordinate deflectors. With this placement, the redistribution of the light illuminance in the light spot on the pupil is minimized when the angular position of the laser beam is varied at the exit of the single-coordinate deflectors. The front focus of a collimating lens 8 is aligned with the center of the field stop or diaphragm 7 to ensure telecentric passage of rays through interferential polarizing beam splitter 9.

An ametropia compensator is schematically depicted as a varifocal group of lenses 10 and 11, adjustable to compensate for the patient's eye ametropia. One of the lenses is mounted on a movable base connected to actuator drive 38. An accommodation controller is schematically depicted as lenses 16 and 17 that constitute a varifocal group of lenses for accommodation control of the patient's eye.

An objective lens 18, at whose focal point the photosensitive surface of a position-sensitive photodetector 19 is located, is intended to form an image of the irradiated retina in the plane of the photosensitive surface of the position-sensitive photodetector. The photosensitive elements of the photodetector are connected through a preamplifier 22 and an analog-to-digital converter 23 to a computer 24. A mirror 39 is movably mounted between the objective lens 18 and the photodetector 19 to optically conjugate the plane of the test-target or plate 20 with the photosensitive surface of the photodetector 19 as well as with the fovea surface. The plate 20 is needed to ensure the fixation of the patient's gaze. Located behind the plate 20 is a light source or radiator 21 serving to illuminate the plate; the light emitted by the radiator is identical in spectral composition to the radiation produced by light source 1, preferably a laser or a polarized light source..

Elements 25 through 30 comprise a microscope whose objective lens consists of lenses 25 and 27 together with mirror 26. A plate 29 with the coordinate-grid is preferably located at the back focal plane of a lens 27, a group of lenses 30, and a beam splitter 28, the front focal point of which coincides with the back focal point of the lens 27, comprise an eyepiece of the microscope. The beam splitter 28 serves to optically couple the retinal plane with the photosensitive plane of a TV camera 32 connected to the computer through a video signal conversion and input board, alternatively termed a frame grabber board, 33.

By means of a mirror 12 provided with an opening, the optical axis of the microscope is aligned with the optical axes of the ray tracing channel (elements 1-11) and the photoelectric arrangement for measuring the transverse ray aberration on the retina (elements 16 - 19).

In a preferred embodiment, four light-emitting diodes (LEDs) 14 are installed in a crosswise configuration in front of the patient's eye. Each LED is preferably located in the same plane as each other LED, at an equal distance from the optical axis and perpendicular with the axis. The microscope and the LEDs comprise a system for the visual and television positioning of the instrument relative to the patient's eye. The microscope is installed so that the front focal plane of lens 25 coincides with the plane, where imaginary or virtual images of the LEDs 14, mirrored by the anterior corneal surface, are located.

Movably mounted between the lens 11 and the LEDs 14 is a mirror 13 which serves to join the optical axes of the instrument and the optical calibration unit 34 - 36. In a preferred embodiment, the optical calibration unit comprises a meniscus or cornea simulator 34, liquid medium or vitreous simulator 35, and retina simulator 36. The retina simulator 36 is preferably movably mounted so that it can be moved along the optical axis by means of actuator or drive 37.

The instant measuring instrument incorporates a computer 24 or like device for controlling the acousto-optic deflector 4, analog-to-digital converter 23, and actuators or drives 37 and 38. The computer 24 or like device or devices may perform additional duties including, for example, mathematical processing and data storage, calculation and display of aberration parameters and the ocular refraction characteristics, as well as providing setting measurement modes and implementation of automatic instrument alignment.

In a preferred embodiment, the instrument functions in the following way. The light beam emitted, for example by laser 1, is expanded, collimated and directed to the acousto-optic deflector 4, which changes its angular position in accordance with the corresponding computer program. The telescopic narrower 5 and 6 reduces the beam thickness to the requisite magnitude. The center of the stop or diaphragm 7, which is being optically coupled with the patient's eye retina due to lenses 8,10 and 11, is a point of angular "swinging" of the beam exiting from the telescopic narrower. Due to its positioning in the front focal plane of the lens 6, the aperture stop or diaphragm AS has its image in the back focal plane of the lens 8 which is aligned with the eye pupil. Thus, the image of the illuminated aperture stop or diaphragm AS on the pupil is the zone of refraction measurement of the patient's eye. Due to the fact that the stop or diaphragm 7 is positioned in the front focal plane of the collimating lens 8, angular swinging of the laser beam with the angle vertex located on the stop or diaphragm 7 is converted into parallel shifting of its optical axis after passing the lens 8.

If the patient's eye is ametropic, the axial movement of the lens 10 (or 11) converts the telecentric beam into a beam which diverges (in the case of myopia) or converges (in the event of hypermetropia) so that the image of the diaphragm 7 is optically coupled with the retina. This also ensures parallelism of the rays reflected by the retina in the zone in front of the beam splitter 9, which is necessary for its normal functioning.

The light entering the eye 15 of the patient is polarized in the plane shown in Figure 1. Only that component of the returning beam depolarized by interaction with the retina is allowed by the beam splitter 9 to pass to the photodetector 19. This protects the photodetector from the polarized light reflected by the surfaces of the lenses 10 and 11 and by the cornea or the eye (as a result of the Fresnel reflex) and which can produce an illuminance incompatible with normal functioning of the instrument.

Lenses 16 and 17 and the objective lens 18 produce an image of the illuminated area of the retina in the plane of the photodetector 19. In Figure 1 the foci locations are designated as follows: $F_3$, $F_5$, $F_6$, $F_8$, $F_{25}$, and $F_{30}$ are points of front foci of the corresponding lenses while $F_2'$, $F_5'$, $F_6'$, $F_{18}'$, and $F_{27}'$ are points of back foci of the lenses.

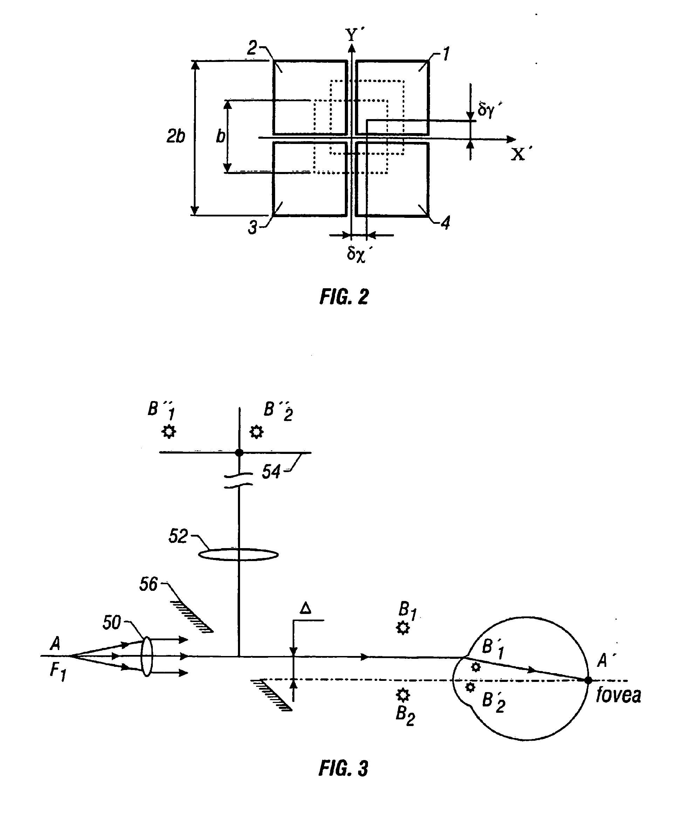

The laser beam is positioned by the computer and the acousto-optic deflector so as to enter the pupil within the requisite refraction measurement zone. If the optical system of the eye has aberration refraction, the light image of the stop or diaphragm 7 on the retina is displaced from the axis, which results in the corresponding displacement of the illuminated zone image on the photosensitive surface of the photodetector and in the variation of electrical signals generated by each of the sensitive elements of the photodetector. If the photodetector 19 is a four-quadrant photodiode as in for example that shown diagrammatically in Figure 2, having quadrants 1 2, 3, & 4, an aberration displacement of the light spot $\delta x$, $\delta y$ on the retina can be given by the formula:

$$\delta x = \frac{\beta}{2} \left[ \frac{(U_1 + U_4) - (U_2 + U_3)}{U_1 + U_2 + U_3 + U_4} \right] \cdot b$$

$$\delta y = \frac{\beta}{2} \left[ \frac{(U_1 + U_2) - (U_3 + U_4)}{U_1 + U_2 + U_3 + U_4} \right] \cdot b$$

where β is the transverse magnification in the plane of the photodetector as related to the plane of the retina, *b* is the size of the light spot in the plane of the photodetector, and $U_1$, $U_2$, $U_3$ and $U_4$ are the photoelectric signals coming from the corresponding photodetector channels.

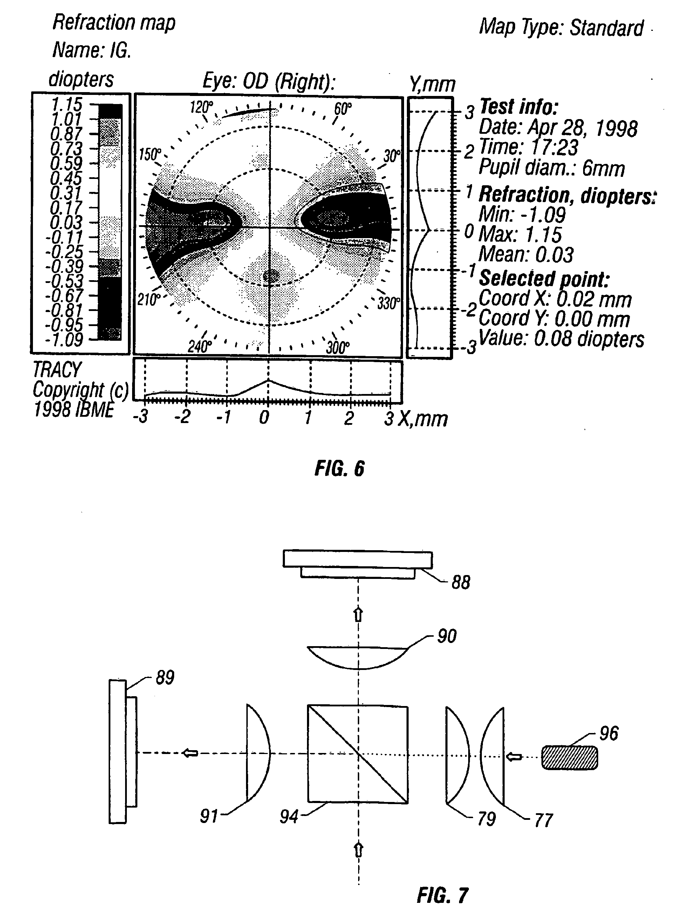

In an alternative preferred embodiment (depicted in Figure 7) light from targeting light source, preferably an infrared light source 96, may be directed through a series of lens 77 and 79 and beam splitter 94, ultimately to an intersection with the optical axis of the instrument and into the patient's eye. The position of a spot of targeting light reflected back from a reflection spot on the fovea of the retina can be determined using reflection beam splitter 94 to direct a first portion of the reflected light from the retina spot through lens 90 to a first linear array photodetector 88 for measuring changes in position only in one direction, for example, in a x-direction. A second portion of the reflected light, substantially identical to the first portion, is directed through lens 91 to a second linear array photodetector 89 for detecting changes in position only in a direction at ninety degrees to the first direction, for example, the y-direction. The change in the x-y position is advantageously measured "directly" for each direction without using the above formula calculation. This is further advantageous because currently available four quadrant photodetectors have a more limited range of aberration that can be detected than currently available linear array photodetectors. For example, a four quadrant detector may cover a 3 diopter aberration range in both directions while two 128 increment linear array photodetectors may cover an aberration range of about 10 diopters in both directions.

In yet another embodiment, the photo detector may be a 2-dimensional or x-y photodetection matrix or a CCD sensory matrix.

Before the refraction measurement process is commenced, the instrument is positioned relative to the patient's eye and the instrument is calibrated using the optical calibration unit 34 - 36.

The principle of operation relating to positioning the instrument in relation to the patient's eye is illustrated in Figure. 3. The collimating system 50 corresponds to the elements 39, 18 and 17, 16 of Figure 1 (if the eye is accommodated at a finite distance). Point A is the light radiator and gaze fixation point and is formed by the elements 20 and 21 in Figure 1. The mirror 56 with an opening corresponds to element 12 of Figure 1. The microscope objective lens 52 is comprised of the elements 25 - 27 of Figure 1 while the microscope objective image plane 54 (Fig. 1) corresponds to the element 29 (Fig. 1). $B_1$ and $B_2$ are light radiators corresponding to the LED 14 of Figure 1. $B'_1$ and $B'_2$ are primary images of the radiators while $B''_1$ and $B''_2$ are secondary images of the radiators.

As can be seen from Figure 3, the fixation of the gaze on the point A, located on the optical axis of the instrument, does not guarantee the coincidence or alignment of the visual axis of the eye and the optical axis of the instrument because the eye sees the point A on the fovea even when $\Delta \neq 0$. The fixation of the gaze on the point A is ensured only when the above axes are parallel.

Taking into account that the largest contribution to the optical power of the eye is made by the anterior surface of the cornea, the visual axis line is assumed to be the line passing through the fovea center and the vertex of center of curvature of the front surface of the cornea. If the radiator $B_1$ is positioned in front of the patient's eye, then, due to Fresnel reflection of the light from the anterior or front surface of the cornea, this surface functioning as a convex mirror, forms an imaginary or virtual image $B'_1$ of the radiator, located symmetrically to the axis in accordance with the laws of geometric optics.

When several radiators, such as for example, $B_1$ and $B_2$, are positioned in front of the patient's eye symmetrically to the optical axis of the instrument (Fig. 3), their secondary images $B''_1$ and $B''_2$ will be shifted in the image plane of the microscope objective lens aside from the axis if $\Delta \neq 0$.

Thus, to make the optical axis of the instrument and the visual axis of the eye coincide, two conditions must be satisfied: the patient's gaze is fixed on the point A and the *images $B''_1$ and $B''_2$* are centrally positioned in relation to the axis of the objective lens 52. The positioning can be checked using the coordinate grid provided on the plate 29 (Fig. 1) or using the monitor screen when the TV channel is utilized, When the image of the eye is aligned at all points with concentric locations on the grid or the TV screen, the measurement controller is armed for taking a spatially resolved set of refraction measurements. The operator can than activate the measurement that can take only a few milliseconds. The measurements are "grabbed" in the grabber board and stored for producing an aberration refraction map as in Figure 6. The measurements can also be activated automatically when the proper alignment is detected. Further, according to one embodiment of the invention, a plurality of measurements can be made sequentially during the occurrence of a predetermined event, such as through a sequence of movement of the eye target from a "near" accommodation distance to a "far"or infinity accommodation distance. A plurality of measurement images can be captured or automatically grabbed and stored over a time period or while any other changes are occurring for which eye measurements might indicate a dynamic change in the refraction of the eye.

The coincidence of the points $B''_1$ and $B''_2$ with the surface or plane 54 is indicative of setting the fixed working distance between the instrument and the eye which is the result of the focusing of the images $B''_1$ and $B''_2$ on the surface 54.

The point of gaze fixation is created by locating the mirror 39 (Fig. 1) on the optical axis of the instrument. The radiators 14' and 14" play the part of the radiators $B_1$ and $B_2$ shown in Fig. 3.

The calibration of the instant aberration refraction instrument may be effected using the optical calibration unit. The optical calibration unit can be made to incorporate known aberrations at the corresponding cornea simulator 34 measurement points. For example, the aberration may be determined by the computer using special optical design programs. If, for example, the front surface of the lens 34 is ellipsoidal, then the aberration refraction at all the points of the pupil is equal to zero.

When an ametropy compensator is used, non-parallel laser beams will enter the optical calibration unit. This will result in a standard aberration of defocusing; to compensate for this aberration, the retina simulator can be moved along the optical axis by means of the actuator 37 to the focus point. Thereby, the fovea can be optically coupled with the retina simulator.

Systematic errors of measurements of the transverse aberration will be evidenced by the deviation of the measurement results from the estimated data. Such determinable systematic errors can be taken into account when measuring actual ocular aberrations.

The calibration by comparison with the optical calibration unit is preferably performed automatically before measuring the ocular aberrations by locating the mirror 13 on the optical axis of the instrument.

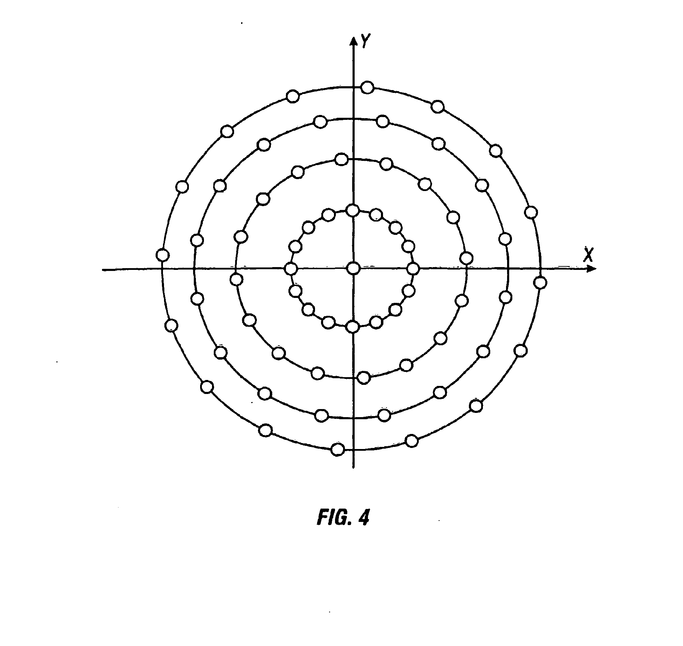

Prior to the ray tracing of the patient's eye, the mirrors 13 and 39 are withdrawn from the light path entering the eye and then passing to the photodetector. The aberration displacement of the image of the light spot on the fovea is measured at a set of points on the cornea corresponding to an ocular ray tracing grid chosen by the operator. An example of a grid or an allocation of measurement points On the pupil is shown in Figure 4.

The data on measurement of the transverse aberrations on the retina $\delta x\,(\rho, \varphi)$ and $\delta y\,(\rho, \varphi)$ are used for further calculations of the coefficients of the Zernike polynomials by means of the least squares method in order to approximate the function of the wave aberration of the eye. The wave aberration function is then used to calculate the local refraction at any point of the pupil. In addition, the approximation makes it possible to determine or reconstruct the nature of local aberration refraction in that small axial zone of the pupil, where it is impossible to make, an accurate direct measurement of refraction.

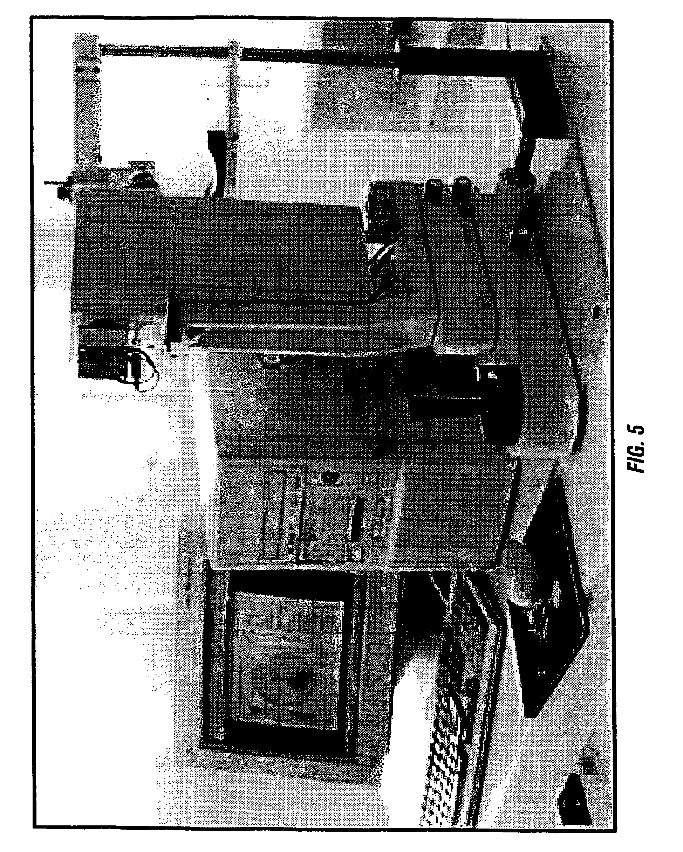

A working embodiment of the present invention is shown in Figure 5. In one experiment conducted using this instrument in which five replicate tests were performed and the results averaged, the laser beam aberration on the retina at 65 points of the pupil was been performed in within 12 milliseconds with no more that 5mW of light radiation entering the eye.

Figure 6 is an example of an ocular aberration refraction map constructed with the use of the instant eye aberration refractometer.

The extremely fast measurement permits the computer control program to cause a plurality of spatially resolved aberration measurements to be made in a very short period of time. The control program in one embodiment automatically activates a plurality of measurements coordinated with a series of adjusted accommodation fixation distances and automatic determination of proper eye alignment to receive a series of data measurements from the retinal spot position detecting channel. A series of refraction measurements for a dynamic eye refraction system is produced. Spatially resolved refraction measurements can be automatically programed and automatically made during a variety of dynamic changes such as varying accommodation or during normal functioning of the eye under a variety of predetermined conditions and internal or external changing conditions.

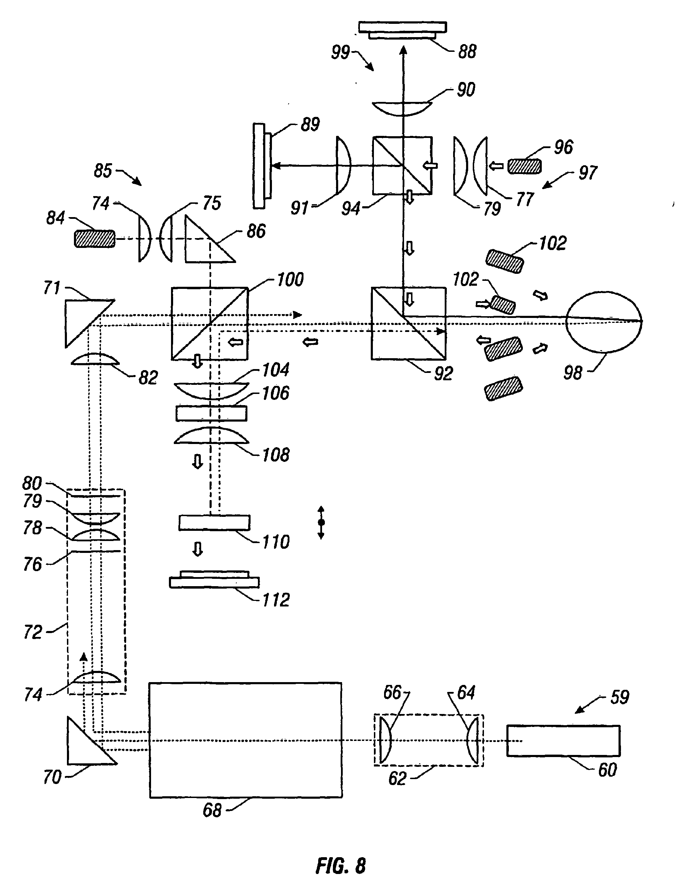

Figure 8 schematically depicts the optical channels of an illustrative example of an aberration refractometer. A spatially defined parallel beam input channel 59 extends from a light source such as a laser or other low diffusion light source up to the eye of a patient 98. In one preferred embodiment a 650λ laser was employed. Along the spatially-defined parallel beam input channel is a cylindrical telescope 62 including two lenses 64 and 66. Light from the cylindrical telescope enters the deflector 68. The deflector 68 is preferably an acousto-optical deflector electronically controlled by a control unit such as a computer. Alternatively, galvanometric mirror deflector or the like could be used. Two coordinate deflectors or angular direction mechanisms may be used as a deflector 68. A reflection mirror or mirror prism 70 reflects the light beam through a telescopic system 72, including preferably, but not necessarily, a lens 74, an entrance aperture 76, lenses 78 and 79 and an exit aperture or field stop 80. The polarized light beam passes from the field stop 80 to collimating lens 82 and is deflected by mirror 71 and passes transparently through beam splitter 100 and interferential beam splitter 92 en route to the eye 98.

Light sources placed in front of the eye are used to align the visual axis of the eye with the optical axis of the instrument. Preferably, a plurality of orthogonally placed LEDs 102, for example, emitting at a λ of 940 mm could be employed. Light produced by LEDs 102 is reflected off the cornea and imaged by camera 112. When the reflected light aligns with preset targeting parameters, the instrument is in the proper alignment and therefore in the permissive mode for firing of the spatially resolved parallel beams formed along channel 59.

Light emitting LEDs combined with light emitted from LED 96 create the eye alignment verification channel. LED 96, preferably an infrared LED, illuminates the eye as it passes through lenses 77 and 79 and through a beam splitter prism 94 and a beam splitter 92 to fall upon the eye 98. The illuminated eye is then ultimately imaged by camera 112 as the image passes through beam splitter prism 92 and is redirected at beam splitter 100 to pass through optical elements 104, 106, 108 and 110 to finally fall upon the CCD camera 112.

A retinal spot position detecting channel 99 is used to detect the position of reflected spots from the retina of eye 98 created by the input channel and includes an interferential polarization beam splitter 92 that directs non-polarized reflected light from the retina of eye 98 to a beam splitter 94 that splits the image directing one component of the image through an optical lens 90 to a "x-coordinate" photodetector 88 and directs another component of the image through optical lens 91 to a "y-coordinate" photodetector 89. Preferably, the orthogonally placed photo detectors 88 and 89 are high resolution linear array photodetectors and the position measurement created on those detectors may be used directly to provide XY coordinates for the measurement of the position of reflected spots on the retina of eye 98. Instead of using linear array detectors, an actual XY matrix photo detector or a CCD detector can be used to replace the beam splitter 94, lenses 90 and 91 and the linear array photodetectors 88 and 89. One benefit of the linear arrays is that they provide for a large range of aberration detection that exceeds the range of a simple quadrant photodetector. For example, a typical quadrant photodetector may be useful for detecting aberrations of a range of about $\pm 3$ diopters while linear arrays can accommodate a range of approximately $\pm 10$ diopters.

A fixation target channel 85 preferably comprises a light source. In a preferred embodiment the light source is a green 565 $\lambda$ LED 84. The light may be transmitted through lenses 74 and 75 and directed by prism 86 and through beam splitter 100 which has wavelength differentiating optical coatings. The fixation target light passes through lenses 104 and 108 and window 106 and is reflected off of the selectively reflective optical filter 110. The fixation target light passes back through elements 108, 106, 104 and is redirected by beam splitter 100 at 90 degrees out toward the eye for the patient to visualize the image as coming from the location of the 110 surface, which can be moved from near fixation to far fixation or adjustable anywhere in between, and this may be used for changing the eye accommodation over a period of time and simultaneously taking a serious of measurements, including spatially resolved aberration refraction measurements as well as pictures on the CCD camera 112. This produces a time lapse imaging of the eye and measurements of the aberration refraction as it cycles through different fixation target distances. The different target fixation distances may be automatically moved or adjusted from near to far using electro mechanical-adjustment means that may be synchronized with the measurements and/or images taken on a time lapse basis.

_References_

I . M. S. Smirnov. Measurement of wave aberration of the eye. Biofizika (Biophysics USER), 6, pp. 776-794, 1961.

2. Van den Brink. Measurement of the geometrical aberrations of the eye. Vision Res. 2, pp. 233-244, 1962.

3. N. M. Sergienko. Oftalmologicheskaya optika (Ophtalmic Optics). Moscow, Meditsina, 1991, 142 pages.

4. R. H. Webb, C. M. Penney, and K. D. Thompson. Measurement of ocular local wavefront distortion with a spatially resolved refractometer. Applied Optics. 31, pp. 3678-3686, 1992.

5. S. G. El Hage and Bemi F. Contribution of the crystalline lens to the spherical aberration of the eye. J. Opt. Soc. Am. 63, pp. 205-211, 1973.

6. J. Liang. A new method to precisely measure the wave aberrations of the human eye with a Hartmann-Shack wave-front sensor, Ph. D. Dissertation, University of Heidelberg, Heidelberg, Germany, 1991.

7. J. Liang, B. Grimm, S. Goelz, and J. F. Bille, Objective measurement of wave aberrations of the human eye with the use of a Hartmann-Shack wave-front sensor. J Opt. Soc. Am. A 11, pp. 1949-1957, 1994.

8. J. Liang and D. R. Williams. Aberrations and retinal image quality of the normal human eye. J Opt. Soc. Am. A 14, pp. 2873-2883, 1997.

9. J. Liang, D. R. Williams, and D. T. Miller. Supernormal vision and high resolution retinal imaging through adaptive optics, J. Opt. Soc. Am., A 14, pp. 2884-2892, 1997.

10. US Patent 5,258,791. Spatially resolved objective autorefractometer, Nov. 2, 1993.

11. T. Seiler, P. J. McDonnell, "Excimer laser photorefractive keratectomy", Surv. of Ophthalm., 40, pp. 89-118, 1995.

**Claims**

1. A device for measuring refraction of an eye as a function of spatial pupil coordinates, comprising:

   a light source (1) producing a probing beam along a path to the eye (15);
   a telescopic system of lenses (2,3,5,6) having an entrance pupil and an exit pupil;
   a two-coordinate deflector (4) consisting of two single-coordinate deflectors separated by a zone;
   a deflection control unit (24);
   an aperture stop (AS);
   a field stop (7);
   a collimating lens (8);
   a position-sensitive photodetector (19) with an objective lens (18) operatively positioned for receiving light reflected from the retina of the eye; and
   a data processing and display unit including a computer (24); and
   wherein the telescopic system is positioned in the probing beam path after the two-coordinate deflector at a distance corresponding to the coincidence of the entrance pupil focal point of the telescopic system and the zone between the single-coordinate deflectors, the aperture stop is placed between the telescopic system lenses at the point of coincidence of their foci, and the field stop is positioned in the plane of the exit pupil of the telescopic system and at the point of location of the front focus of the collimating lens;
   **characterized in that**:

an interferential polarizing beam splitter (9) is positioned along the path to the eye between the collimating lens and the eye;

a varifocal ametropia compensator comprising a varifocal group of lenses (10,11) adjustable to compensate for eye ametropia is positioned behind the interferential polarization beam splitter between the position-sensitive surface of the photodetector and the eye in the path of light scattered by the retina; and

an eye accommodation controller comprising a varifocal group of lenses (16, 17) is positioned in front of the interferential polarizing beam splitter between the position sensitive surface of the photodetector and the eye.

2. The measuring device according to claim 1, wherein use is made of a laser and/or infrared light source for producing the probing beam.

3. The measuring device according to claims 1 or 2, wherein the light source producing the probing beam is a polarized or a laser light probing beam being directed by the interferential polarization beam splitter and further passes only non-polarized light as scattered back from the retina to the photodetector.

4. The measuring device according to claim 3, further comprising:

an optical axis bending mirror (39) movable into and out of the light path; and

a gaze fixation plate (20) having a test pattern thereon, wherein the mirror and the gaze fixation plate are optically coupled with the photodetector and are located between the photodetector and the objective lens and wherein the eye accommodation controller serves to form a test pattern image visible to the eye at a distance corresponding to a variably selected accommodation.

5. The measuring device according to claim 3 or 4, further comprising:

one or more point light sources (14) positioned in front of the eye along the probing light beam path;

an optical axis bending mirror (13) movable into and out of the probing laser beam path for measuring the retinal standard characteristics and withdrawn therefrom before measuring eye aberration refraction;

an optical calibration unit including a cornea simulator (34), a vitreous simulator (35) and an axially movable or stationary retina simulator (36), wherein the mirror is positioned in the probing light beam path between the varifocal lens (11) and the eye, which mirror optically links the laser light beam path to the optical calibration unit.

6. The measuring device according to claim 5, wherein the optical axis bending mirror (13) further comprises an optical coating which permits selective transmission of light such that it need not be removed from the probing laser beam path when measuring eye aberration refraction.

7. The measuring device according to any one of claims 3 to 6, further comprising:

a viewing channel for verifying the alignment of the device with the eye including:

the one or more point light sources (14) positioned in front of the eye;

a microscope having an objective consisting of lenses (25, 27) and mirror (26), a beam splitter (28), a plate with a coordinate-grid (29) a group of lenses (30);

a TV or optoelectronic photodetector (32) serving to display an image of the eye and produce a signal enabling the measurement of the ocular characteristics when the optical axis of the measuring device and the visual axis of the patient's eye coincide, the TV or optoelectronic photodetector optically coupled to the microscope by the beam splitter (28); and

a mirror (12) with an opening that aligns the optical axis of the microscope with the optical axis comprising a ray tracing channel (1-11) and the optical axis comprising the photoelectric arrangement for measuring retinal aberration (16-19).

8. The measuring device according to claim 7, wherein the channel produces an automatic permission signal via an electronic circuitry or a computer electronically linked to said channel and contains a program recognizing said automatic permission signal and directing measurement of the ocular characteristics directly upon achieving preset or derived alignment criteria according to the permission signal.

9. The measuring device according to claims 7 or 8, wherein the point light source comprises laser and/or infrared light radiation sources.

10. The measuring device according to any one of claims 1 to 9 wherein the photodetector is a quadrant photodetector, orthogonally placed linear array photodetectors, XY matrix photodetectors, or CCD detector.

**Patentansprüche**

1. Vorrichtung zum Messen der Refraktion eines Auges in Abhängigkeit von räumlichen Pupillenkoordinaten, umfassend:

eine Lichtquelle (1), die einen Sondierungsstrahl entlang eines Pfades zum Auge (15) erzeugt;
ein teleskopisches System von Linsen (2, 3, 5, 6), das eine Eintrittspupille und eine Austrittspupille aufweist;
einen Zwei-Koordinaten-Ablenker (4), der aus zwei durch eine Zone getrennten Einzel-Koordinaten-Ablenkern besteht;
eine Ablenkungs-Steuereinheit (24);
eine Aperturblende (AS);
einen Feldblende (7);
eine Sammellinse (8);
einen positionsempfindlichen Fotodetektor (19) mit einer Objektivlinse (18), die im Betrieb angeordnet ist, um aus der Retina des Auges reflektiertes Licht zu empfangen; sowie
eine Datenverarbeitungs- und Anzeigeeinheit, die einen Rechner (24) einschließt; und
wobei das teleskopische System im Sondierungsstrahlpfad nach dem Zwei-Koordinaten-Ablenker in einem Abstand entsprechend der Koinzidenz des Eintrittspupillen-Brennpunkts des teleskopischen Systems und der Zone zwischen den Einzel-Koordinaten-Ablenkern angeordnet ist, die Aperturblende zwischen den Linsen des teleskopischen Systems am Koinzidenzpunkt ihrer Brennpunkte angeordnet ist, und die Feldblende in der Ebene der Austrittspupille des teleskopischen Systems und am Positionierungspunkt des vorderen Brennpunkts der Sammellinse angeordnet ist;
**dadurch gekennzeichnet, dass**:

ein Interferenz-Polarisationsstrahlteiler (9) entlang des Pfades zum Auge zwischen der Sammellinse und dem Auge angeordnet ist;
ein Variofocal-Ametropie-Kompensator, umfassend eine Variofocal-Gruppe von Linsen (10, 11), die einstellbar sind, um die Augen-Ametropie zu kompensieren, hinter dem Interferenz-Polarisationsstrahlteiler zwischen der positionsempfindlichen Oberfläche des Fotodetektors und dem Auge im Pfad des von der Retina gestreuten Lichts angeordnet ist; und
eine Augen-Akkomodations-Steuerung, umfassend eine Variofocal-Gruppe von Linsen (16, 17) vor dem Interferenz-Polarisationsstrahlteiler zwischen der positionsempfindlichen Oberfläche des Fotodetektors und dem Auge angeordnet ist.

2. Messvorrichtung nach Anspruch 1, wobei von einer Laser-und/oder Infrarotlichtquelle Gebrauch gemacht wird, um den Sondierungsstrahl zu erzeugen.

3. Messvorrichtung nach den Ansprüchen 1 oder 2, wobei die Lichtquelle, die den Sondierungsstrahl erzeugt, ein polarisierter oder ein Laserlicht-Sondierungsstrahl ist, wobei er vom Interferenz-Polarisationsstrahlteiler gelenkt wird und weiter nur nicht-polarisiertes Licht durchlässt, wie es von der Retina zum Fotodetektor zurückgestreut wird.

4. Messvorrichtung nach Anspruch 3, weiter umfassend:

einen Optikachsen-Biegespiegel (39), der in den und aus dem Lichtpfad beweglich ist; und
eine Blickfixierungsplatte (20) mit einem Testmuster darauf, wobei der Spiegel und die Blickfixierungsplatte optisch mit dem Fotodetektor gekoppelt sind und zwischen dem Fotodetektor und der Objektivlinse angeordnet sind, und wobei die Augen-Akkomodations-Steuerung dazu dient, ein für das Auge sichtbares Testmusterbild in einem Abstand zu bilden, der einer veränderlich ausgewählten Akkomodation entspricht.

5. Messvorrichtung nach Anspruch 3 oder 4, weiter umfassend:

eine oder mehrere Punktlichtquellen (14), die vor dem Auge entlang des Sondierungslichtstrahlpfades angeordnet sind;

einen Optikachsen-Biegespiegel (13), der in den und aus dem Sondierungslaserstrahlpfad beweglich ist, um die Retina-Standardeigenschaften zu messen, und vor dem Messen der Augen-Aberrationsrefraktion daraus zurückgezogen wird;

eine optische Kalibrierungseinheit, die einen Augenhornhaut-Simulator (34), einen Glaskörper-Simulator (35) und einen axial beweglichen oder stationären Retina-Simulator (36) einschließt, wobei der Spiegel im Sondierungslichtstrahlpfad zwischen der Variofocal-Linse (11) und dem Auge angeordnet ist, welcher Spiegel den Laserlichtstrahlpfad mit der optischen Kalibrierungseinheit verbindet.

6. Messvorrichtung nach Anspruch 5, wobei der Optikachsen-Biegespiegel (13) weiter eine optische Beschichtung umfasst, die einen selektiven Lichtdurchlass gestattet, so dass er nicht aus dem Sondierungslaserlichtstrahlpfad entfernt werden braucht, wenn die Augen-Aberrationsrefraktion gemessen wird.

7. Messvorrichtung nach einem der Ansprüche 3 bis 6, weiter umfassend:

einen Betrachtungskanal zur Bestätigung der Ausrichtung der Vorrichtung mit dem Auge, einschließend:

die eine oder mehreren, vor dem Auge angeordneten Punktlichtquellen (14);
ein Mikroskop mit einem Objektiv, das aus Linsen (25, 27) und einem Spiegel (26) besteht, einem Strahlteiler (28), einer Platte mit einem Koordinatengitter (29), einer Gruppe von Linsen (30);
einen Fernseh- oder Optoelektronik-Fotodetektor (32), der dazu dient, ein Bild des Auges darzustellen und ein Signal zu erzeugen, das die Messung der Augen-Eigenschaften ermöglicht, wenn die optische Achse der Messvorrichtung und die Sehachse des Auges des Patienten zusammenfallen, wobei der Fernseh- oder Optoelektronik-Fotodetektor durch den Strahlteiler (28) optisch mit dem Mikroskop gekoppelt ist; und
einen Spiegel (12) mit einer Öffnung, der die Optikachse des Mikroskops mit der einen Strahlengangverfolgungskanal (1-11) umfassenden Optikachse und der die fotoelektrische Anordnung zum Messen der Retina-Aberration (16-19) umfassenden Optikachse ausrichtet.

8. Messvorrichtung nach Anspruch 7, wobei der Kanal ein automatisches Erlaubnissignal über eine elektronische Schaltung oder einen elektronisch mit dem besagten Kanal verbundenen Rechner erzeugt und ein Programm enthält, das das automatische Erlaubnissignal erkennt und die Messung der Augen-Eigenschaften direkt beim Erreichen von voreingestellten oder abgeleiteten Ausrichtungskriterien gemäß dem Erlaubnissignal leitet.

9. Messvorrichtung nach den Ansprüchen 7 oder 8, bei der die Punktlichtquelle Laser- und/oder Infrarotlichtstrahlungsquellen umfasst.

10. Messvorrichtung nach einem der Ansprüche 1 bis 9, wobei der Fotodetektor ein Quadranten-Fotodetektor, orthogonal platzierte Lineararray-Fotodetektoren, XY-Matrix-Fotodetektoren oder CCD-Detektor ist.

**Revendications**

1. Dispositif pour mesurer la réfraction d'un oeil en fonction de coordonnées spatiales de pupille, comprenant :

une source de lumière (1) produisant un faisceau de sondage le long d'un trajet vers l'oeil (15) ;
un système télescopique de lentilles (2, 3, 5, 6) ayant une pupille d'entrée et une pupille de sortie ;
un déflecteur à deux coordonnées (4) consistant en deux déflecteurs à coordonnée unique séparés par une zone ;
une unité de commande de déflexion (24) ;
un diaphragme d'ouverture (AS) ;
un diaphragme de champ (7) ;
une lentille de collimation (8) ;
un photodétecteur sensible à la position (19) avec une lentille de focalisation (18) positionnée fonctionnellement pour recevoir la lumière réfléchie par la rétine de l'oeil ; et
une unité de traitement et d'affichage de données comprenant un ordinateur (24) ; et
dans lequel le système télescopique est positionné dans le trajet du faisceau de sondage après le déflecteur à deux coordonnées à une distance correspondant à la coïncidence du foyer de la pupille d'entrée du système télescopique et de la zone entre les déflecteurs à coordonnée unique, le diaphragme d'ouverture est placé entre les lentilles du système télescopique au point de coïncidence de leurs foyers, et le diaphragme de champ est positionné dans le plan de la pupille de sortie du système télescopique et au point d'emplacement du foyer avant de la lentille de collimation ;

**caractérisé en ce que** :

un diviseur de faisceau de polarisation interférentiel (9) est positionné le long du trajet vers l'oeil entre la lentille de collimation et l'oeil ;

un compensateur d'amétropie à foyer progressif comprenant un groupe de lentilles à foyer progressif (10, 11) ajustable pour compenser une amétropie de l'oeil est positionné derrière le diviseur de faisceau de polarisation interférentiel entre la surface sensible à la position du photodétecteur et l'oeil dans le trajet de la lumière diffusée par la rétine ; et

un contrôleur d'accommodation de l'oeil comprenant un groupe de lentilles à foyer progressif (16, 17) est positionné devant le diviseur de faisceau de polarisation interférentiel entre la surface sensible à la position du photodétecteur et l'oeil.

2. Dispositif de mesure selon la revendication 1, dans lequel une source de lumière laser et/ou infrarouge est utilisée pour produire le faisceau de sondage.

3. Dispositif de mesure selon la revendication 1 ou 2, dans lequel la source de lumière produisant le faisceau de sondage est un faisceau de sondage de lumière polarisée ou laser dirigé par le diviseur de faisceau de polarisation interférentiel et, en outre, ne laisse passer que la lumière non polarisée telle que diffusée de retour de la rétine vers le photodétecteur.

4. Dispositif de mesure selon la revendication 3, comprenant en outre :

un miroir de repliement d'axe optique (39) pouvant être déplacé dans et hors du trajet de lumière ; et

une plaque de fixation du regard (20) sur laquelle se trouve un motif de test, dans lequel le miroir et la plaque de fixation du regard sont couplés optiquement avec le photodétecteur et sont situés entre le photodétecteur et la lentille de focalisation et dans lequel le contrôleur d'accommodation de l'oeil sert à former une image de motif de test visible par l'oeil à une distance correspondant à une accommodation sélectionnée de manière variable.

5. Dispositif de mesure selon la revendication 3 ou 4, comprenant en outre :

une ou plusieurs sources de lumière ponctuelle (14) positionnée devant l'oeil le long du trajet du faisceau de lumière de sondage ;

un miroir de repliement d'axe optique (13) pouvant être déplacé dans et hors du trajet du faisceau laser de sondage pour mesurer les caractéristiques standard rétiniennes et retiré de celui-ci avant de mesurer la réfraction d'aberration de l'oeil ;

une unité d'étalonnage optique comprenant un simulateur de cornée (34), un simulateur de vitré (35) et un simulateur de rétine (36) mobile axialement ou fixe, dans lequel le miroir est positionné dans le trajet du faisceau de lumière de sondage entre la lentille à foyer progressif (11) et l'oeil, lequel miroir relie optiquement le trajet du faisceau de lumière laser à l'unité d'étalonnage optique.

6. Dispositif de mesure selon la revendication 5, dans lequel le miroir de repliement d'axe optique (13) comprend en outre un revêtement optique qui permet une transmission sélective de la lumière de sorte qu'il ne doit pas être retiré du trajet du faisceau laser de sondage lors de la mesure de la réfraction d'aberration de l'oeil.

7. Dispositif de mesure selon l'une quelconque des revendications 3 à 6, comprenant en outre :

un canal d'observation pour vérifier l'alignement du dispositif avec l'oeil comprenant :

lesdites une ou plusieurs sources de lumière ponctuelle (14) positionnées devant l'oeil ;

un microscope comportant un objectif consistant en des lentilles (25, 27) et un miroir (26), un diviseur de faisceau (28), une plaque avec une grille de coordonnées (29), un groupe de lentilles (30) ;

un photodétecteur TV ou optoélectronique (32) servant à afficher une image de l'oeil et à produire un signal permettant la mesure des caractéristiques oculaires lorsque l'axe optique du dispositif de mesure et l'axe visuel de l'oeil du patient coïncident, le photodétecteur TV ou optoélectronique étant couplé optiquement au microscope par le diviseur de faisceau (28) ; et

un miroir (12) avec une ouverture qui aligne l'axe optique du microscope avec l'axe optique comprenant un canal de suivi de rayon (1-11) et l'axe optique comprenant l'agencement photoélectrique pour mesurer une aberration rétinienne (16-19).

8. Dispositif de mesure selon la revendication 7, dans lequel le canal produit un signal d'autorisation automatique par l'intermédiaire d'éléments de circuit électroniques ou d'un ordinateur liés électroniquement audit canal et contient un programme reconnaissant ledit signal d'autorisation automatique et dirigeant la mesure des caractéristiques oculaires directement lors de l'obtention de critères d'alignement prédéterminés ou déduits conformément au signal d'autorisation.

9. Dispositif de mesure selon la revendication 7 ou 8, dans lequel la source de lumière ponctuelle comprend des sources de rayonnement de lumière laser et/ou infrarouge.

10. Dispositif de mesure selon l'une quelconque des revendications 1 à 9, dans lequel le photodétecteur est un photodétecteur à quadrants, des photodétecteurs à réseau linéaire placés orthogonalement, des photodétecteurs à matrice XY ou un détecteur à CCD.

**FIG. 1**

**FIG. 2**

**FIG. 3**

16

**FIG. 4**

FIG. 5

Refraction map
Name: IG.

Map Type: Standard

diopters

Eye: OD (Right):

Y,mm

1.15
1.01
0.87
0.73
0.59
0.45
0.31
0.17
0.03
-0.11
-0.25
-0.39
-0.53
-0.67
-0.81
-0.95
-1.09

120°    60°

150°    30°

210°    330°

240°    300°

3

2

1

0

-1

-2

-3

**Test info:**
Date: Apr 28, 1998
Time: 17:23
Pupil diam.: 6mm
**Refraction, diopters:**
Min: -1.09
Max: 1.15
Mean: 0.03
**Selected point:**
Coord X: 0.02 mm
Coord Y: 0.00 mm
Value: 0.08 diopters

TRACY
Copyright (c)
1998 IBME

-3  -2  -1  0  1  2  3 X,mm

**FIG. 6**

**FIG. 7**

**FIG. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5258791 A

### Non-patent literature cited in the description

- **M. S. Smirnov.** Measurement of wave aberration of the eye. *Biofizika (Biophysics USER),* 1961, vol. 6, 776-794
- **Van den Brink.** Measurement of the geometrical aberrations of the eye. *Vision Res.,* 1962, vol. 2, 233-244
- **N. M. Sergienko.** Oftalmologicheskaya optika (Ophtalmic Optics). *Meditsina,* 1991, 142
- **R. H. Webb ; C. M. Penney ; K. D. Thompson.** Measurement of ocular local wavefront distortion with a spatially resolved refractometer. *Applied Optics,* 1992, vol. 31, 3678-3686
- **S. G. El Hage ; Bemi F.** Contribution of the crystalline lens to the spherical aberration of the eye. *J. Opt. Soc. Am.,* 1973, vol. 63, 205-211
- **J. Liang.** A new method to precisely measure the wave aberrations of the human eye with a Hartmann-Shack wave-front sensor. *Ph. D. Dissertation,* 1991
- **J. Liang ; B. Grimm ; S. Goelz ; J. F. Bille.** Objective measurement of wave aberrations of the human eye with the use of a Hartmann-Shack wave-front sensor. *J Opt. Soc. Am. A,* 1994, vol. 11, 1949-1957
- **J. Liang ; D. R. Williams.** Aberrations and retinal image quality of the normal human eye. *J Opt. Soc. Am. A,* 1997, vol. 14, 2873-2883
- **J. Liang ; D. R. Williams ; D. T. Miller.** Supernormal vision and high resolution retinal imaging through adaptive optics. *J. Opt. Soc. Am.,* 1997, vol. A 14, 2884-2892
- **T. Seiler ; P. J. McDonnell.** Excimer laser photorefractive keratectomy. *Surv. of Ophthalm.,* 1995, vol. 40, 89-118